# EUROPEAN PATENT APPLICATION

(11) **EP 1 813 651 A1**
(43) Date of publication of application: **01.08.2007**
(21) Application number: 05807091.3
(22) Date of filing: 18.11.2005
(51) Int. Cl.: C09B 57/00, C07D 403/08, C07D 401/08, C07D 417/08, G02B 5/20

(54) **OPTICAL FILTER**

(30) Priority: 19.11.2004 JP 2004335700
(71) Applicant: Kyowa Hakko Chemical Co., Ltd., Tokyo 103-0022 (JP)
(72) Inventor: YAMANO, Junzo. c/oYokkaichi Research Laboratories, Yokkaichi-shi, Mie 510-8502, (JP); UKAI, Katsumi. c/oYokkaichi Research Laboratories, Yokkaichi-shi, Mie 510-8502, (JP); KINUGASA, Motoharu. c/oYokkaichi Research Laboratories, Yokkaichi-shi, Mie 510-8502, (JP)
(74) Representative: Casalonga, Axel
(86) International application number: PCT/JP2005/021249
(87) International publication number: WO 2006/054700

(57) **Abstract**

The present invention provides optical filters comprising a squarylium compound represented by General Formula (I): 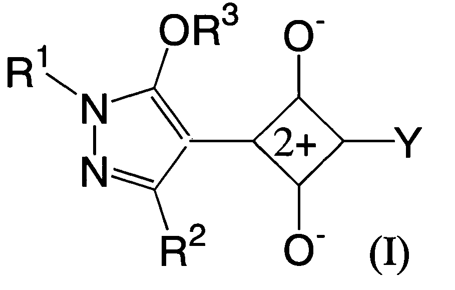 [wherein R¹ and R² may be the same or different and each represents a hydrogen atom, an alkyl group optionally having substituent(s) or the like, R³ represents a hydrogen atom or an alkyl group optionally having substituent(s), and Y represents a group represented by General Formula (A): 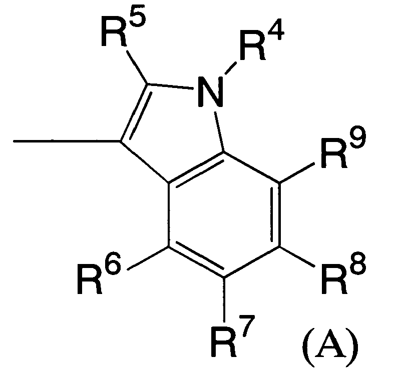 (wherein R⁴ represents a hydrogen atom, an alkyl group optionally having substituent(s) or the like, R⁵ represents a hydrogen atom, a halogen atom or the like, and R⁶, R⁷, R⁸ and R⁹ may be the same or different and each represents a hydrogen atom, a halogen atom, an alkyl group optionally having substituent(s) or the like)] and the like.

## Description

### Technical Field

The present invention relates to optical filters comprising a squarylium compound.

### Background Art

When external lights reflect to glass covers and the like of imaging products such as electronic display devices, hand-held gaming devices, image screens; show cases, clocks, and the like, visibilities of the said products decrease. As a countermeasure to the visibility decrease, a method using filters that reduce transmission of lights at a central wavelength of average relative luminosity curve, which is around 555nm, or 530 to 580 nm, is known (refer to Patent Documents 1 to 3).

When the above method is applied to electronic display devices such as plasma panel displays, color qualities of the displays improve because unnecessary long-wavelength side of broad green fluorescent material emission is omitted. However, when other visible parts of the light are absorbed, or when absorbing wavelength regions are very broad, etc., there are possibilities that the said method may notably decrease light transmittance necessary for electronic display devices.
Patent Document 1: Japanese Published Unexamined Patent Application No. 1995-307133
Patent Document 2: Japanese Published Unexamined Patent Application No. 1998-204304
Patent Document 3: Japanese Published Unexamined Patent Application No. 2003-167118

### Disclosure of the Invention

### Problems to be Solved by the Invention

An object of the present invention is to provide optical filters which improve color quality of electronic display devices, etc., and the like.

### Means for Solving the Problem

The present invention provides the following (1) to (14):
(1) An optical filter comprising a squarylium compound represented by General Formula (I): 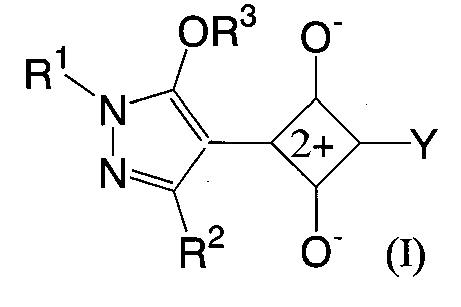 {wherein R¹ and R² may be the same or different and each represents a hydrogen atom, an alkyl group optionally having substituent(s), an aralkyl group optionally having substituent(s), an aryl group optionally having substituent(s), a heterocyclic group optionally having substituent(s), or an amino group optionally having substituent(s), R³ represents a hydrogen atom or an alkyl group optionally having substituent(s), and Y represents a group represented by General Formula (A): 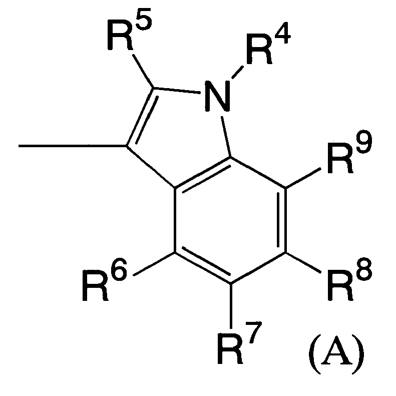 [wherein R⁴ represents a hydrogen atom, an alkyl group optionally having substituent(s), an aralkyl group optionally having substituent(s), an aryl group optionally having substituent(s), or a heterocyclic group optionally having substituent(s), R⁵ represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, an amino group optionally having substituent(s), or a heterocyclic group optionally having substituent(s), and R⁶, R⁷, R⁸, and R⁹ may be the same or different and each represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, or a heterocyclic group optionally having substituent(s), or R⁶ and R⁷, R⁷ and R⁸, or R⁸ and R⁹ are combined to form a methylenedioxy group or are combined together with the two adjacent carbon atoms thereto, respectively, to form a hydrocarbon ring optionally having substituent(s)], a group represented by General Formula (B): 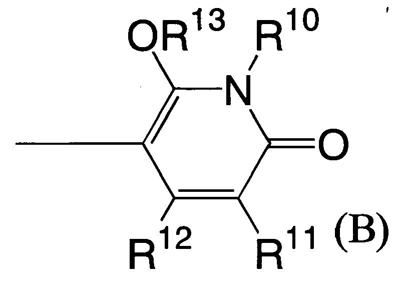 [wherein R¹⁰ and R¹² may be the same or different and each represents a hydrogen atom, an alkyl group optionally having substituent(s), an aralkyl group optionally having substituent(s), an aryl group optionally having substituent(s), or a heterocyclic group optionally having substituent(s), R¹¹ represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, an amino group optionally having substituent(s), a carbamoyl group optionally having substituent(s), a carboxyl group, an alkoxycarbonyl group optionally having substituent(s), a sulfamoyl group optionally having substituent(s), an alkylsulfonyl group optionally having substituent(s), a sulfo group, a sulfomethyl group, or a heterocyclic group optionally having substituent(s), and R¹³ represents a hydrogen atom or an alkyl group optionally having substituent(s), or R¹¹ and R¹² may be combined together with two adjacent carbon atoms thereto, respectively, to form a hydrocarbon ring optionally having substituent(s)] or a group represented by General Formula (C): 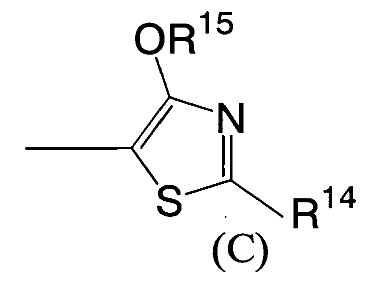 [wherein R¹⁴ represents a hydrogen atom, a halogen atom, a hydroxyl group, an alkyl group optionally having substituent(s), an alkoxyl group optionally having substituent(s), an aralkyl group optionally having substituent(s), an aryl group optionally having substituent(s), an amino group optionally having substituent(s), or a heterocyclic group optionally having substituent(s), and R¹⁵ represents a hydrogen atom, or an alkyl group optionally having substituent(s)]}.
(2) An optical filter comprising a squarylium compound represented by General Formula (Ia): 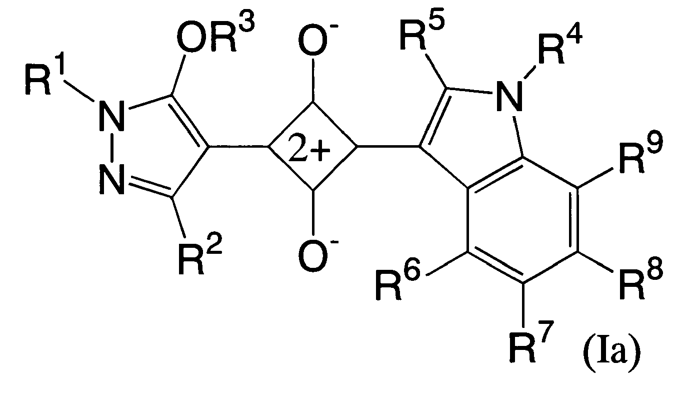 (wherein R¹, R², R³, R⁴, R⁵, R⁶, R⁷, R⁸, and R⁹ have the same definitions as described above, respectively).
(3) An optical filter comprising a squarylium compound represented by General Formula (Ib): 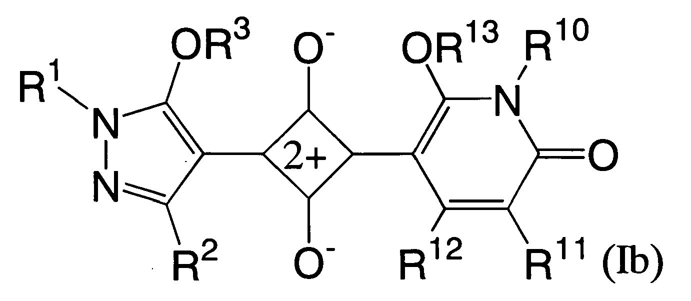 (wherein R¹, R², R³, R¹⁰, R¹¹, R¹² and R¹³ have the same definitions as described above, respectively).
(4) An optical filter comprising a squarylium compound represented by General Formula (Ic): 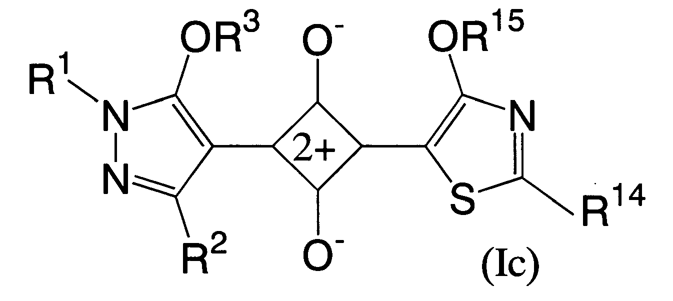 (wherein R¹, R², R³, R¹⁴ and R¹⁵ have the same definitions as described above, respectively).
(5) The optical filter according to any one of (1) to (4), which has an absorption maximum in a wavelength region of 530 nm to 580 nm.
(6) The optical filter according to any one of (1) to (5), wherein the optical filter is a filter for plasma display devices.
(7) A squarylium compound represented by General Formula (I): 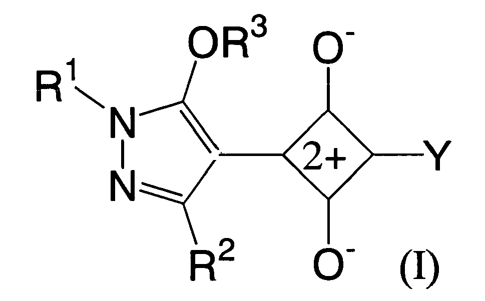 (wherein R¹, R², R³ and Y have the same definitions as described above, respectively).
(8) A squarylium compound represented by General Formula (Ia): 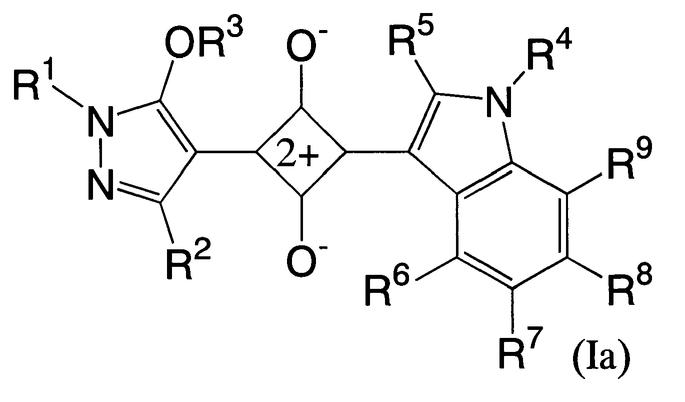 (wherein R¹, R², R³, R⁴, R⁵, R⁶, R⁷, R⁸ and R⁹ have the same definitions as described above, respectively).
(9) A squarylium compound represented by General Formula (Ib): 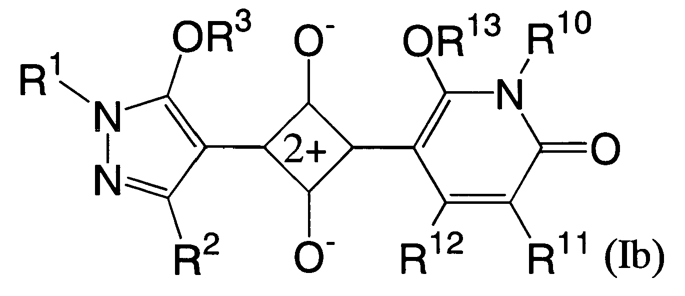 (wherein R¹, R², R³, R¹⁰, R¹¹, R¹², and R¹³ have the same definitions as described above, respectively).
(10) A squarylium compound represented by General Formula (Ic): 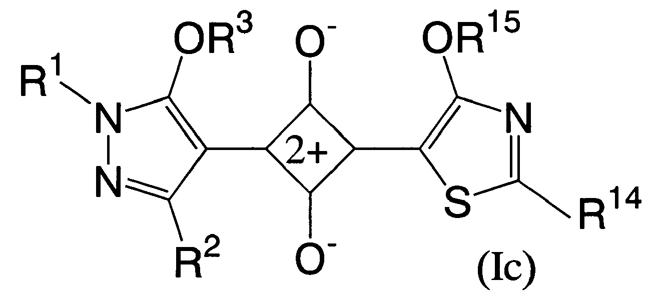 (wherein R¹, R², R³, R¹⁴ and R¹⁵ have the same definitions as described above, respectively).
(11) The squarylium compound according to the above (8), wherein R³ represents a hydrogen atom and R⁴ represents a hydrogen atom, an alkyl group or an alkoxyalkoxy-substituted alkyl group.
(12) The squarylium compound according to the above (9), wherein R³ and R¹³ each represents a hydrogen atom and R¹¹ represents a cyano group.
(13) The squarylium compound according to the above (10), wherein R³ represents a hydrogen atom, R¹⁴ represents an amino group optionally having substituent(s), or a heterocyclic group optionally having substituent(s), and R¹⁵ represents an alkyl group.
(14) The squarylium compound according to any one of the above (7) to (13), wherein R¹ represents a tert-butyl group or a phenyl group.

### Effect of the Invention

The present invention provides optical filters which improve color quality of the electronic display devices, etc., and the like.

### Best Mode for Carrying Out the Invention

Hereinafter, the compound represented by General Formula (I) is referred to as Compound (I). Compounds with other formula numbers are also expressed in the same manner.

In the definition of each group in the general formulae, examples of the alkyl group, an alkyl moiety in the alkoxyl group, alkoxycarbonyl group, and alkylsufonyl group, and three alkyl moieties in the alkoxyalkoxy-substituted alkyl group include, for example, linear or branched alkyl groups having one to six carbon atoms and cyclic alkyl groups having three to eight carbon atoms, specifically, such as a methyl group, an ethyl group, a propyl group, an isopropyl group, a butyl group, an isobutyl group, a sec-butyl group, a tert-butyl group, a pentyl group, an isopentyl group, a 2-methylbutyl group, a tert-pentyl group, a hexyl group, a cyclopropyl group, a cyclobutyl group, a cyclopentyl group, a cyclohexyl group, a cycloheptyl group, and a cyclooctyl group.

Examples of the aralkyl group include aralkyl groups having seven to fifteen carbon atoms, specifically, such as a benzyl group, a phenethyl group, a phenylpropyl group, and a naphtylmethyl group.

Examples of the aryl group include a phenyl group, a naphthyl group, an anthryl group and the like.

The halogen atom includes a fluorine atom, a chlorine atom, a bromine atom, and an iodine atom.

Examples of a heterocyclic ring in the heterocyclic group include aromatic heterocyclic rings and alicyclic heterocyclic rings.

Examples of the aromatic heterocyclic rings include 5-to 7-membered monocyclic aromatic heterocyclic rings containing at least one atom selected from a nitrogen atom, an oxygen atom, and a sulfur atom; fused bicyclic or tricyclic aromatic heterocyclic rings containing at least one atom selected from a nitrogen atom, an oxygen atom, and a sulfur atom wherein 3- to 8-membered rings are fused; and the like. More specific examples thereof are a pyridine ring, a pyrazine ring, a pyrimidine ring, a pyridazine ring, a quinoline ring, an isoquinoline ring, a phthalazine ring, a quinazoline ring, a quinoxaline ring, a naphthyridine ring, a cinnoline ring, a pyrrole ring, a pyrazole ring, an imidazole ring, a triazole ring, a tetrazole ring, a thiophene ring, a furan ring, a thiazole ring, an oxazole ring, an indole ring, an isoindole ring, an indazole ring, a benzimidazole ring, a benzotriazole ring, a benzothiazole ring, a benzoxazole ring, a purine ring, a carbazole ring, and the like.

Examples of the alicyclic heterocyclic rings include 5-to 7- membered monocyclic alicyclic heterocyclic rings containing at least one atom selected from a nitrogen atom, an oxygen atom and a sulfur atom; fused bicyclic or tricyclic alicyclic heterocyclic rings containing at least one atom selected from a nitrogen atom, an oxygen atom and a sulfur atom wherein 3- to 8- membered rings are fused; and the like. More specific examples thereof are a pyrrolidine ring, a piperidine ring, a piperazine ring, a morpholine ring, a thiomorpholine ring, a homopiperidine ring, a homopiperazine ring, a tetrahydropyridine ring, a tetrahydroquinoline ring, a tetrahydroisoquinoline ring, a tetrahydrofuran ring, a tetrahydropyran ring, a dihydrobenzofuran ring, a tetrahydrocarbazole ring, and the like.

Examples of the hydrocarbon ring wherein R⁶ and R⁷, R⁷ and R⁸, R⁸ and R⁹, or R¹¹ and R¹² are combined together with two adjacent carbon atoms thereto, respectively, include, for example, unsaturated hydrocarbon rings having five to ten carbon atoms such as a cyclopentene ring, a cyclohexene ring, a cycloheptene ring, a cyclooctene ring, a benzene ring, and a naphthalene ring.

Examples of the substituent(s) of the alkyl group, alkoxyl group, alkoxycarbonyl group, and alkylsulfonyl group include, for example, one to three substituents which may be the same or different. More specific examples thereof are a hydroxyl group, a carboxyl group, a cyano group, a halogen atom, an alkoxyl group, an alkoxyalkoxyl group, an amino group optionally having substituent(s), a nitro group, and the like. The halogen atom and the alkoxyl group have the same definitions as described above, respectively. The two alkoxy moieties of the alkoxyalkoxyl group have the same definitions as the above alkoxyl group, respectively.

Examples of the substituent(s) of the aralkyl group, aryl group, heterocyclic group, and hydrocarbon ring wherein R⁶ and R⁷, R⁷ and R⁸, R⁸ and R⁹, or R¹¹ and R¹² are combined together with two adjacent carbon atoms thereto, respectively, include, for example, one to five substituents which may be the same or different, specifically, such as a hydroxyl group, a carboxyl group, a halogen atom, an alkyl group, an alkoxyl group, a nitro group, and an amino group optionally having substituent(s). The halogen atom, the alkyl group and the alkoxyl group have the same definitions as described above, respectively.

Examples of the substituent(s) of the amino group, carbamoyl group, and sulfamoyl group include, for example, one or two substituent(s) which may be the same or different, specifically, such as an alkyl group, an aralkyl group, and an aryl group. The alkyl group, the aralkyl group, and the aryl group have the same definitions as described above, respectively.

For example, Compound (Ia), Compound (Ib) and Compound (Ic) can be prepared in the following manner. 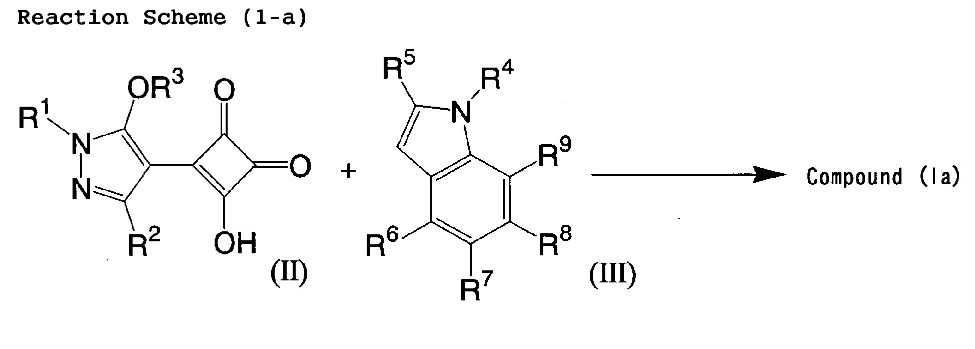 (wherein R¹, R², R³, R⁴, R⁵, R⁶, R⁷, R³ and R⁹ have the same definitions as described above, respectively). 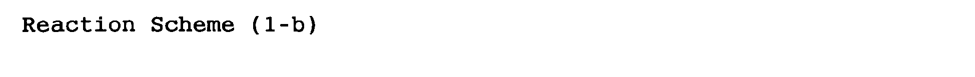 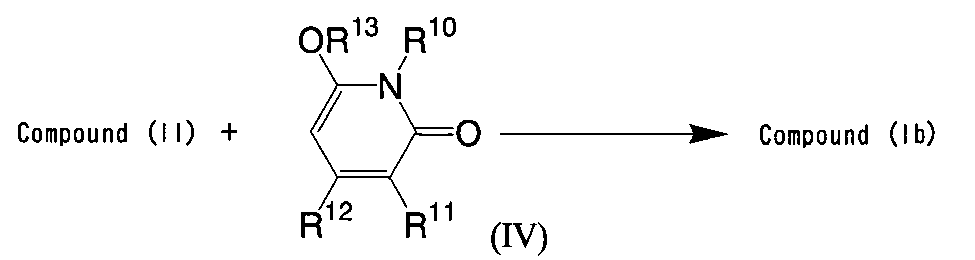 (wherein R¹⁰, R¹¹, R¹² and R¹³ have the same definitions as described above, respectively). 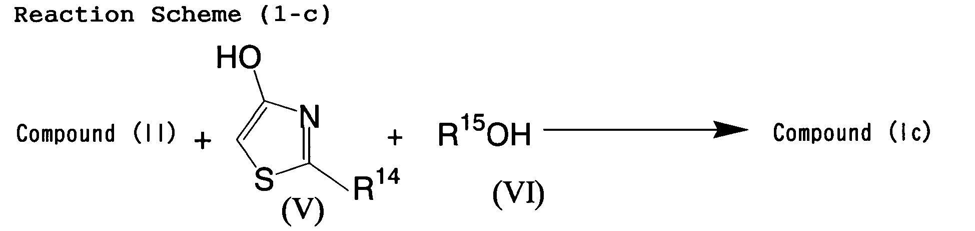 (wherein R¹⁴ and R¹⁵ have the same definitions as described above, respectively).

### Reaction Scheme (1-a), (1-b) and (1-c)

The Compound (II) can be prepared in a similar manner to a known method (e.g., WO 01/44233).

The Compound (III) can be prepared in a similar manner to a known method (e.g., Chem. Rev., 63, 371-401, (1963)) or as a commercially available product.

The Compound (IV) can be prepared in a similar manner to a known method (e.g., Japanese Published Unexamined Patent Application No. 1976-37919 and the like) or as a commercially available product.

The Compound (V) can be prepared in a similar manner to a known method (e.g., J. Indian Chem. Soc., 69(6), 314-317(1992)).

The Compound (Ia) can be obtained by reacting the Compound (II) with 1- to 5-fold moles of the Compound (III) at a temperature of 80°C to 130°C for one to 24 hours in a solvent.

The Compound (Ib) can be obtained by reacting the Compound (II) with 1- to 5-fold moles of the Compound (IV) at a temperature of 80°C to 130°C for one to 24 hours in a solvent.

The Compound (Ic) can be obtained by reacting the Compound (II) with 1- to 5-fold moles of the Compound (V) and 1-fold moles to an excessive amount of the Compound (VI) at a temperature of 80°C to 130°C for one to 24 hours in a solvent.

Examples of the solvent include an alcohol solvent such as ethanol, propanol, isopropyl alcohol, butanol, or octanol; a mixed solvent of the alcohol solvent with benzene, toluene, or xylene, and the like.

After the reaction, if necessary, the desired compound may be purified by a procedure generally used in synthetic organic chemistry (such as column chromatography, recrystallization, or washing with a solvent and the like).

Preferred examples of the Compound (I) are illustrated below. In the structural formulae , Me represents a methyl group; Et represents an ethyl group; Pr represents a propyl group; Bu represents a buthyl group; and Ph represents a phenyl group.

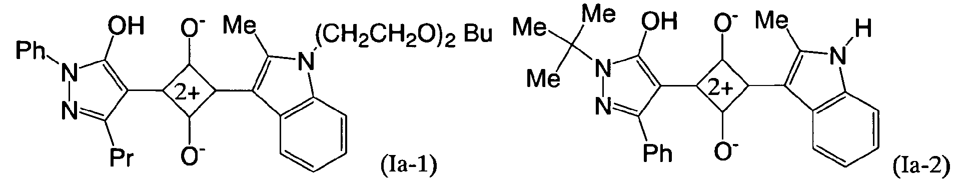

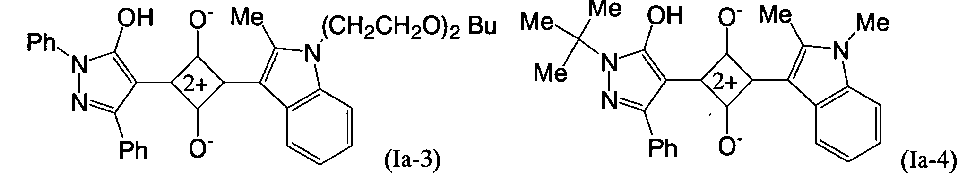

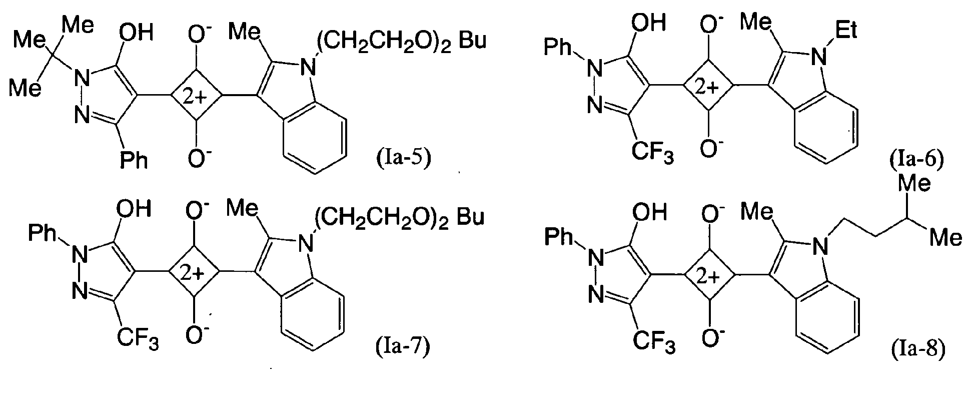

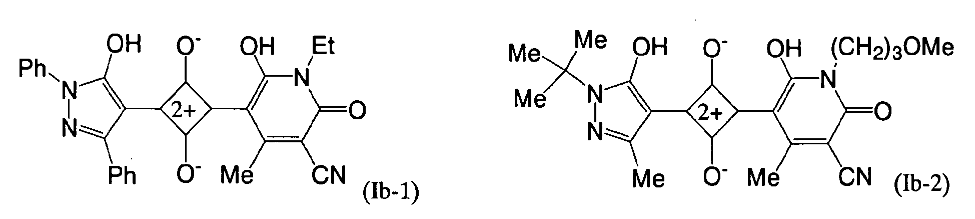

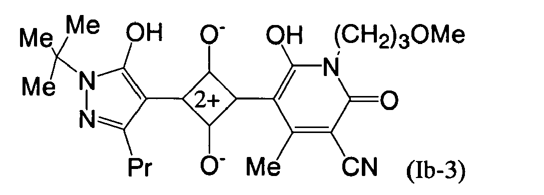

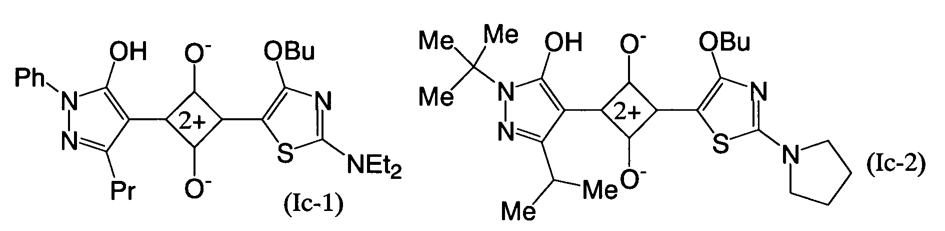

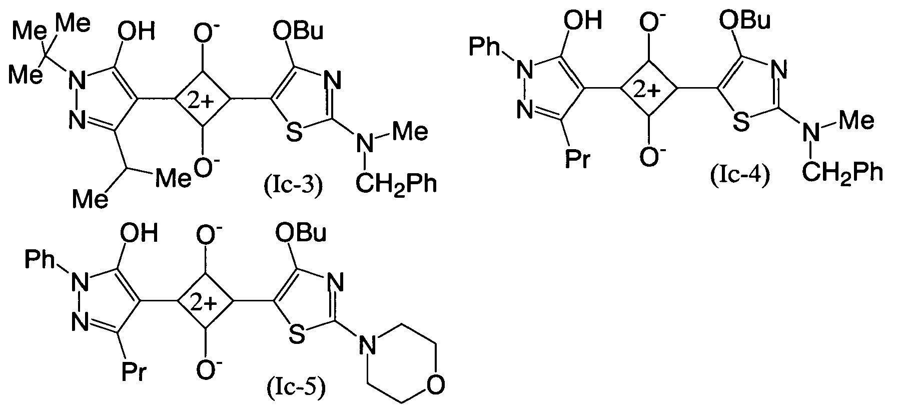

The Compound (I) used for the optical filter of the present invention preferably has an absorption maximum in an absorption region of 530 nm to 580 nm in a chloroform solution. The Compound (I) used for the optical filter of the present invention also preferably has logarithm of a molar extinction coefficient of 4.5 or more, and more preferably 4.8 or more.

The optical filter of the present invention preferably has an absorption maximum in an absorption region of 530 to 580 nm.

The optical filter of the present invention is preferably produced by applying a coating composition containing the Compound (I) to an optically transparent substrate, and evaporating the organic solvent. If necessary, another optically transparent substrate may be laminated.

The coating composition may be prepared by dissolving a solution of an organic solvent containing the Compound (I) with a binder in the organic solvent.

Examples of the organic solvent include ethers such as dimethoxyethane, methoxyethoxyethane, tetrahydrofuran, and dioxane; ketones such as acetone, methylethylketone, methylisobutylketone, and cyclohexanone; and aromatic hydrocarbons such as benzene, toluene, xylene, and monochlorobenzene; and the like. These organic solvents are preferably used in an amount 10 to 3000-fold by weight to the Compound (I).

Examples of the binder include a polyester resin, a polycarbonate resin, a polyacrylic acid resin, a polystyrene resin, a poly(vinyl chloride) resin, a poly(vinyl acetate) resin and the like. The binder is preferably used in an amount 10- to 500-fold by weight to the Compound (I).

The optically transparent substrate is not specifically limited, as long as it is an optically transparent resin or glass having low absorption and scattering. Examples of the resin include a polyester resin, a polycarbonate resin, a poly(acrylic acid) resin, a polystyrenic resin, a poly(vinyl chloride) resin, a poly(vinyl acetate) resin and the like.

The coating composition containing the Compound (I) can be applied to the optically transparent substrate according to a known coating procedure, such as bar coating, spraying, roll coating, or dipping (e.g., U.S. Patent No. 2,681,294 and the like).

The Compound (I) has a high solubility in an organic solvent and is suitable for a method of preparing the optical filter using the above coating composition.

The optical filter of the present invention may also be prepared by directly dissolving or dispersing the Compound (I) in a resin constituting an optically transparent substrate, forming the solution or dispersion into a film, and, if necessary, laminating the film with other optically transparent substrates at one or both sides thereof.

The film formed from the Compound (I) preferably has a half maximum full-width (a width of wavelength region indicating half of the absorbance in an absorption maximum wavelength) of 100 nm or less and more preferably 80 nm or less in an absorption maximum wavelength. The film formed from the Compound (I) having a sufficient transmittance in red luminescence region and having a transmittance of 80 % or more at 615 nm is preferred.

Typical lighting apparatuses for households such as three band phosphor type fluorescent lamps or white fluorescent lamps have high emission intensity in a region where visibility of human is high, which is from 530 to 580 nm. The optical filters of the present invention are able to reduce reflection of external lights by absorbing the external lights in the said wavelength region.

The optical filters of the present invention are able to prevent glare caused by reflection of external lights such as lightings, and are able to prevent decrease in visibility caused by reflection of surrounding scenery, while having excellent contrast or color quality (By suppressing transmittance of yellow-green region, the color quality of green especially improves).

The optical filter of the present invention can be used for, for example, goggles; lens sheets; polarizing plates; optical components such as fiber optics; transparent covers such as showcases, frames, photo frames, glass for pachinko (vertical pinball), hand-held gaming devices, and clocks; electronic display devices such as cathode-ray tube displays, liquid crystal displays, plasma display panels, organic electroluminescence panels, field emission displays, and rear projection display devices; and the like. Among them, the filter can be used preferably for electronic display devices, especially for plasma display panels.

The present invention will be illustrated in further detail with reference to the following Examples and Test Examples.

### EXAMPLE 1: Preparation of Compound (Ia-1)

To a mixed solvent of 3 ml of butanol and 3 ml of toluene, 1.00 g of 3-hydroxy-4-(5-hydroxy-l-phenyl-3-propylpyrazol-4-yl)cyclobutene-1,2-dione and 1.02 g of 1-butoxyethoxyethyl-2-methylindole were added, and the mixture was reacted at reflux temperature for 2 hours. Then, the reaction mixture was cooled to 20°C to 30°C, and 6 ml of methanol was added thereto. The precipitated solid was collected by filtration to obtain 1.57 g of Compound (Ia-1). ¹H-NMR d(CDCl₃)ppm: 0.88(3H, t, J=7.3Hz), 1.05(3H, t, J=7.3Hz), 1.27-1.33(2H, m), 1.45-1.50(2H, m), 1.75-1.81(2H, m), 2.87(3H, t, J=7.4Hz), 3.05(3H, s), 3.42-3.49(4H, m), 3.78(3H, t, J=5.5Hz), 4.25(3H, t, J=5.5Hz), 7.18-7.29(4H, m), 7.39-7.43(2H, m), 7.87-7.90(2H, m), 8.68-8.70(1H, m).

### EXAMPLE 2: Preparation of Compound (Ia-2)

To a mixed solvent of 3 ml of butanol and 3 ml of toluene, 1.00 g of 3-hydroxy-4-(1-tert-butyl-5-hydroxy-3-phenylpyrazol-4-yl)cyclobutene-1,2-dione and 0.46 g of 2-methylindole were added and the mixture was reacted at reflux temperature for 2 hours. Then, the reaction mixture was cooled to 20°C to 30°C and 6 ml of methanol was added. The precipitated solid was collected by filtration to obtain 1.16 g of Compound (Ia-2).
¹H-NMR d(CDCl₃)ppm: 1.65(9H, s), 2.99(3H, s), 7.22-7.24(3H, m), 7.42-7.43(3H, m), 7.72-7.74(2H, m), 8.68-8.70(1H, m), 8.9(1H, brs).

### EXAMPLE 3: Preparation of Compound (Ia-3)

By treating 2.91 g of toluene solution containing 1.00 g of 3-hydroxy-4-(5-hydroxy-1,3-diphenylpyrazol-4-yl)cyclobutene-1,2-dione and 1.02 g of 1-butoxyethoxyethyl-2-methylindole in a similar manner to Example 1, 1.32 g of Compound (Ia-3) was obtained.
¹H-NMR d(CDCl₃)ppm: 0.89(3H, t, J=7.3Hz), 1.26-1.36(2H, m), 1.46-1.53(2H, m), 3.08(3H, s), 3.33-3.37(2H, m), 3.44-3.52(4H, m), 3.83(3H, t, J=5.4Hz), 4.33(3H, t, J=5.4Hz), 7.27-7.30(3H, m), 7.44-7.50(5H, m), 7.80-7.82(2H, m), 8.00-8.01(2H, m), 8.71-8.73(1H, m).

### EXAMPLE 4: Preparation of Compound (Ia-4)

By treating 1.00 g of 3-hydroxy-4-(1-tert-butyl-5-hydroxy-3-phenylpyrazol-4-yl)cyclobutene-1,2-dione and 0.51 g of 1,2-dimethylindole in a similar manner to Example 2, 1.31 g of Compound (Ia-4) was obtained.
¹H-NMR d(CDCl₃)ppm: 1.66(9H, s), 3.07(3H, s), 3.71(3H, s), 7.23-7.29(3H, m), 7.44-7.47(3H, m), 7.73-7.75(2H, m), 8.79-8.82(1H, m).

### EXAMPLE 5: Preparation of Compound (Ia-5)

By treating 2.78 g of toluene solution containing 1.00 g of 3-hydroxy-4-(1-tert-butyl-5-hydroxy-3-phenylpyrazol-4-yl)cyclobutene-1,2-dione and 0.97 g of 1-butoxyethoxyethyl-2-methylindole in a similar manner to Example 1, 1.51 g of Compound (Ia-5) was obtained.
¹H-NMR δ (CDCl₃)ppm: 0.89(3H, t, J=7.5Hz), 1.28-1.34(2H, m), 1.46-1.56(2H, m), 1.65(9H, s), 3.11(3H, s), 3.35(3H, t, J=6.6Hz), 3.44-3.51(4H, m), 3.83(3H, t, J=5.5Hz), 4.34(3H, t, J=5.5Hz), 7.26-7.29(3H, m), 7.44-7.47(3H, m), 7.73-7.75(2H, m), 8.80-8.82(1H, m).

### EXAMPLE 6: Preparation of Compound (Ib-1)

To a mixed solvent of 10 ml of butanol and 10 ml of toluene, 1.00 g of 3-hydroxy-4-(5-hydroxy-1,3-diphenylpyrazol-4-yl)cyclobutene-1,2-dione and 0.59 g of 3-cyano-1-ethyl-6-hydroxy-4-methyl-2-pyridone were added, and the mixture was reacted at reflux temperature for 3 hours. Then, the reaction mixture was cooled to 0°C to 10°C, and 40 ml of methanol was added thereto. The precipitated solid was collected by filtration to obtain 0.90 g of Compound (Ib-1).
¹H-NMR δ (CDCl₃)ppm: 1.20(3H, t, J=7.0Hz), 2.83(3H, s), 4.03(2H, q, J=7.0Hz), 7.37-7.52(6H, m), 7.59-7.62(2H, m), 7.87-7.89(2H, m), 13.6(1H, br).

### EXAMPLE 7: Preparation of Compound (Ib-2)

By treating 1.00 g of 3-hydroxy-4-(1-tert-butyl-5-hydroxy-3-methylpyrazol-4-yl)cyclobutene-1,2-dione and 0.98 g of 3-cyano-6-hydroxy-1-methoxypropyl-4-methyl-2-pyridone in a similar manner to Example 6, 1.09 g of Compound (Ib-2) was obtained.
¹H-NMR δ (CDCl₃)ppm: 1.60(9H, s), 1.87-1.94(2H, m), 2.40(3H, s), 2.79(3H, s), 3.31(3H, s), 3.45(2H, t, J=6.0Hz), 4.13(2H, t, J=7.2Hz), 12.7(1H, br).

### EXAMPLE 8: Preparation of Compound (Ib-3)

By treating 1.00 g of 3-hydroxy-4-(1-tert-butyl-5-hydroxy-3-propylpyrazol-4-yl)cyclobutene-1,2-dione and 0.88 g of 3-cyano-6-hydroxy-1-methoxypropyl-4-methyl-2-pyridone in a similar manner to Example 6, 0.72 g of Compound (Ib-3) can be obtained.
¹H-NMR (CDCl₃)ppm: 0.99(2H, t, J=7.4Hz), 1.60(9H, s), 1.62-1.70(2H, m), 1.87-1.95(2H, m), 2.71-2.75(2H, m), 2.79(3H, s), 3.32(3H, s), 3.46(2H, t, J=6.0Hz), 4.13(2H, t, J=7.3Hz), 12.9(1H, br).

### EXAMPLE 9: Preparation of Compound (Ic-1)

To a mixed solvent of 20 ml of butanol and 20 ml of toluene, 1.00 g of 3-hydroxy-4-(1-phenyl-5-hydroxy-3-propylpyrazol-4-yl)cyclobutene-1,2-dione and 1.14 g of 1-diethylamino-4-hydroxythiazole were added, and the mixture was reacted at reflux temperature for 11 hours. Then, the reaction mixture was cooled to 20°C to 30°C, and the precipitated solid was collected by filtration to obtain 0.70 g of Compound (Ic-1).
¹H-NMR d(CDCl₃)ppm: 1.00(3H, t, J=7.5Hz), 1.04(3H, t, J=7.5Hz), 1.3(6H, br), 1.53-1.59(2H, m), 1.74-1.87(4H, m), 2.87-2.91(2H, m), 3.5(2H, br), 3.79-3.82(2H, m), 3.8(2H, br), 4.54(2H, t, J=6.4Hz), 7.23(1H, t, J=7.4Hz), 7.41(2H, t, J=7.8Hz), 7.85(2H, d, J=8.0Hz).

### EXAMPLE 10: Preparation of Compound (Ic-2)

By treating 1.00 g of 3-hydroxy-4-(1-tert-butyl-5-hydroxy-3-isopropylpyrazol-4-yl)cyclobutene-1,2-dione and 0.96 g of 2-(1-pyrrolidino)-4-hydroxythiazole in a similar manner to Example 9, 0.14 g of Compound (Ic-2) was obtained.
¹H-NMR d(CDCl₃)ppm: 0.99(3H, t, J=7.4Hz), 1.26(6H, d, J=6.8Hz), 1.55(2H, br), 1.56(9H, s), 1.85(2H, br), 2.09-2.17(4H, m), 3.44(2H, br), 3.5-3.6(1H, m), 3.80(2H, br), 4.58(2H, t, J=6.6Hz)

### EXAMPLE 11: Preparation of Compound (Ia-6)

To 10 ml of butanol solution containing 5.94 g of 3-hydroxy-4-[(5-hydroxy-3-trifluoromethyl-1-phenyl)pyrazol-4-yl]cyclobutene-1,2-dione, 10 ml of toluene solution containing 2.92 g of 1-ethyl-2-methylindole was added, and the mixture was reacted at a temperature of 90°C to 100°C for 3 hours. Then, the reaction mixture was cooled to 20°C to 30°C, and the precipitated solid was collected by filtration to obtain 6.81 g of Compound (Ia-6).
¹H-NMR δ (CDCl₃)ppm: 1.46 (3H, t, J=7.2Hz), 3.17 (3H, s), 4.24 (2H, q, J=7.2Hz), 7.25-7.42 (4H, m), 7.44-7.52 (2H, m), 7.86-7.93 (2H, m), 8.82 (1H, m).

### EXAMPLE 12: Preparation of Compound (Ia-7)

To 10 ml of butanol solution containing 3.24 g of 3-hydroxy-4-[(5-hydroxy-3-trifluoromethyl-1-phenyl)pyrazol-4-yl]cyclobutene-1,2-dione, 10 ml of toluene solution containing 2.75 g of 1-butoxyethoxyethyl-2-methylindole was added, and the mixture was reacted at a temperature of 100°C to 110°C for 8 hours. Then, the reaction mixture was cooled to 20°C to 30°C, and 25 mL of methanol was added thereto. The precipitated solid was collected by filtration to obtain 2.52 g of Compound (Ia-7).
¹H-NMR δ (CDCl₃) ppm: 0.88 (3H, t, J=7.2Hz), 1.26-1.38 (2H, m), 1.48-1.60 (2H, m), 3.22 (3H, s), 3.32-3.36 (2H, m), 3.45-3.55 (4H, m), 3.85-3.90 (2H, m), 4.38-4.42 (2H, m), 7.25-7.38 (4H, m), 7.55-7.62 (2H, m), 7.86-7.93 (2H, m), 8.84 (1H, m).

### EXAMPLE 13: Preparation of Compound (Ia-8)

To 8 ml of butanol solution containing 3.94 g of 3-hydroxy-4-[(5-hydroxy-3-trifluoromethyl-1-phenyl)pyrazol-4-yl]cyclobutene-1,2-dione, 8 ml of toluene solution containing 2.45 g of 1-(3-methylbutyl)-2-methylindole was added, and the mixture was reacted at a temperature of 100°C to 110°C for 3 hours. Then, the reaction mixture was cooled to 20°C to 30°C, and the precipitated solid was collected by filtration to obtain 4.23 g of Compound (Ia-8).
¹H-NMR δ (CDCl₃)ppm: 1.06 (6H, d, J=7.2Hz), 1.66-1.88 (3H, m), 3.16 (3H, s), 4.15-4.20 (2H, m), 7.25-7.40 (4H, m), 7.45-7.50 (2H, m), 7.86-7.90 (2H, m), 8.78 (1H, m).

### EXAMPLE 14: Preparation of Compound (Ic-3)

By treating 0.50 g of 3-hydroxy-4-[4-(1-tert-butyl-5-hydroxy-3-isopropyl)pyrazoyl]cyclobutene-1,2-dione and 0.40 g of 2-(N-benzyl-N-methyl)amino-4-hydroxythiazole in a similar manner to Example 9, 0.45 g of Compound (Ic-3) was obtained.
¹H-NMR d(CDCl₃) ppm: 0.99(3H, t, J=7.3Hz), 1.26(6H, d, J=6.8Hz), 1.52-1.58(2H, m), 1.56(9H, s), 1.86(2H, m), 3.09(2H, m), 3.2-3.6 (3H, br), 4.55(2H, t, J=6.6Hz), 4.96(2H, br), 7.26-7.27(3H, m), 7.36-7.41(2H, m).

### EXAMPLE 15: Preparation of Compound (Ic-4)

By treating 0.50 g of 3-hydroxy-4-[4-(1-phenyl-5-hydroxy-3-propyl)pyrazoyl]cyclobutene-1,2-dione and 0.37 g of 2-(N-benzyl-N-methyl)amino-4-hydroxythiazole in a similar manner to Example 9, 0.45 g of Compound (Ic-4) was obtained.
¹H-NMR d(CDCl₃)ppm: 1.00(3H, t, J=7.6Hz), 1.04(3H, t, J=7.4Hz), 1.53-1.59(2H, m), 3.09(2H, m), 3.2-3.6 (3H, br), 4.55(2H, t, J=6.6Hz),1.74-1.79(2H, m), 4.54(2H, t, J=6.4Hz), 4.96(2H, br), 7.22-7.27(4H, m,), 7.36-7.44(4H, m), 7.85(2H, d, J=8.0Hz).

### EXAMPLE 16: Preparation of Compound (Ic-5)

By treating 0.50 g of 3-hydroxy-4-[4-(1-phenyl-5-hydroxy-3-propyl)pyrazoyl]cyclobutene-1,2-dione and 0.32 g of 2-morpholino-4-hydroxythiazole in a similar manner to Example 9, 0.28 g of Compound (Ic-5) was obtained.
¹H-NMR d(CDCl₃)ppm: 0.99(3H, t, J=7.6Hz), 1.03(3H, t, J=7.3Hz), 1.52-1.58(2H, m), 1.73-1.79(2H, m), 1.85(2H, m), 2.88(2H, m), 3.76-3.86(8H, m), 4.53(2H, t, J=6.5Hz), 7.24(1H, t, J=7.4Hz), 7.40-7.44(2H, m), 7.85(2H, d J=7.8Hz).

### TEST EXAMPLE 1

The absorption maximum wavelength (λmax) and logarithm of a molar extinction coefficient (logε) of Compounds (Ia-1) to (Ia-8), Compounds (Ib-1) to (Ib-3), and Compounds (Ic-1) to (Ic-5) in a chloroform solution were measured (800 to 300 nm) using Spectorophotometer [UV-4000(Hitachi Co., Ltd.)]. The results are shown in Table 1.

**Table 1: Spectroscopic property of squarylium compounds**

| Compound | Spectroscopic property (Chloroform solution) | |
|---|---|---|
| | λmax (nm) | logε |
| Ia-1 | 545 | 5.0 |
| Ia-2 | 544 | 5.0 |
| Ia-3 | 553 | 5.0 |
| Ia-4 | 548 | 5.1 |
| Ia-5 | 550 | 5.1 |
| Ia-6 | 555 | 5.0 |
| Ia-7 | 555 | 5.0 |
| Ia-8 | 555 | 5.0 |
| Ib-1 | 555 | 5.0 |
| Ib-2 | 546 | 5.0 |
| Ib-3 | 548 | 5.0 |
| Ic-1 | 552 | 5.1 |
| Ic-2 | 551 | 5.2 |
| Ic-3 | 554 | 5.2 |
| Ic-4 | 555 | 5.2 |
| Ic-5 | 557 | 5.2 |

### TEST EXAMPLE 2

Each of a 0.5 percent by weight solution of Compound (Ia-5) or Compound (Ib-2) in dimethoxyethane, a 1.0 percent by weight solution of Compound (Ic-1) in dimethoxyethane and a 20 percent by weight solution of a polyester resin [VYLON 200 (a product of TOYOBO Co., Ltd.)] in dimethoxyethane were mixed at a ratio of 7:2, and the mixture was applied to a glass substrate using a spin coater, and dried to yield a coating film. The absorption maximum wavelength, the half maximum full-width, and the transmittance at 615 nm of the film were measured(800 to 300 nm) using Spectorophotometer [UV-4000(Hitachi Co., Ltd.)]. The results are shown in Table 2.

**Table 2: Absorption maximum wavelengths, half maximum full-widths, and transmittances at 615 nm of squarylium compounds in a film**

| Compound | Absorption maximum wavelength (nm) | Half maximum full-width (nm) | Transmittance at 615 nm (%) |
|---|---|---|---|
| Ia-5 | 557 | 67 | 95 or more |
| Ib-2 | 552 | 68 | 95 or more |
| Ic-1 | 562 | 98 | 90 or more |

These results show that the optical filters using Compounds (Ia-5), (Ib-2) or (Ic-1) of the present invention can selectively shield the light having such a wavelength as to reduce the color quality.

### Industrial Applicability

The present invention provides optical filters which improve color quality of electric display devices, etc., and the like.

## Claims

1. An optical filter comprising a squarylium compound represented by General Formula (I): 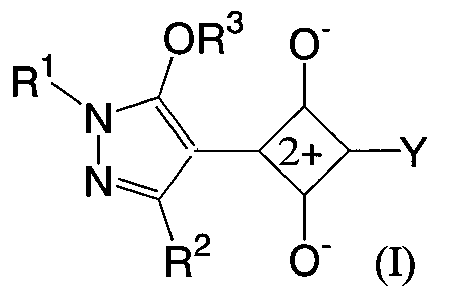 {wherein R¹ and R² may be the same or different and each represents a hydrogen atom, an alkyl group optionally having substituent(s), an aralkyl group optionally having substituent(s), an aryl group optionally having substituent(s), a heterocyclic group optionally having substituent(s), or an amino group optionally having substituent(s), R³ represents a hydrogen atom or an alkyl group optionally having substituent(s), and Y represents a group represented by General Formula (A): 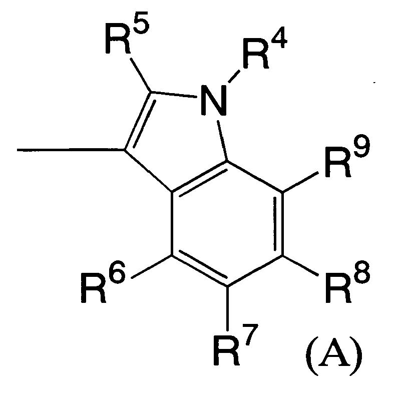 [wherein R⁴ represents a hydrogen atom, an alkyl group optionally having substituent(s), an aralkyl group optionally having substituent(s), an aryl group optionally having substituent(s), or a heterocyclic group optionally having substituent(s), R⁵ represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, an amino group optionally having substituent(s), or a heterocyclic group optionally having substituent(s), and R⁶, R⁷, R⁸, and R⁹ may be the same or different and each represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, or a heterocyclic group optionally having substituent(s), or R⁶ and R⁷, R⁷ and R⁸, or R⁸ and R⁹ are combined to form a methylenedioxy group or are combined together with two adjacent carbon atoms thereto, respectively, to form a hydrocarbon ring optionally having substituent(s)], a group represented by General Formula (B): 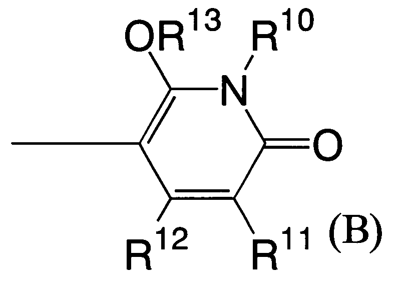 [wherein R¹⁰ and R¹² may be the same or different and each represents a hydrogen atom, an alkyl group optionally having substituent(s), an aralkyl group optionally having substituent(s), an aryl group optionally having substituent(s), or a heterocyclic group optionally having substituent(s), R¹¹ represents a hydrogen atom, a halogen atom, an alkyl group optionally having substituent(s), an alkoxyl group optionally having substituent(s), an aralkyl group optionally having substituent(s), an aryl group optionally having substituent(s), a nitro group, a cyano group, a hydroxyl group, an amino group optionally having substituent(s), a carbamoyl group optionally having substituent(s), a carboxyl group, an alkoxycarbonyl group optionally having substituent(s), a sulfamoyl group optionally having substituent(s), an alkylsulfonyl group optionally having substituent(s), a sulfo group, a sulfomethyl group, or a heterocyclic group optionally having substituent(s), and R¹³ represents a hydrogen atom or an alkyl group optionally having substituent(s), or R¹¹ and R¹² may be combined together with two adjacent carbon atoms thereto, respectively, to form a hydrocarbon ring optionally having substituent(s)] or a group represented by General Formula (C): 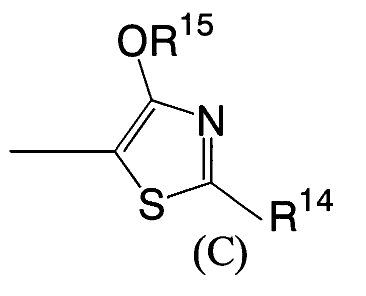 [wherein R¹⁴ represents a hydrogen atom, a halogen atom, a hydroxyl group, an alkyl group optionally having substituent(s), an alkoxyl group optionally having substituent(s), an aralkyl group optionally having substituent(s), an aryl group optionally having substituent(s), an amino group optionally having substituent(s), or a heterocyclic group optionally having substituent(s), and R¹⁵ represents a hydrogen atom, or an alkyl group optionally having substituent(s)]}.

2. An optical filter comprising a squarylium compound represented by General Formula (Ia): 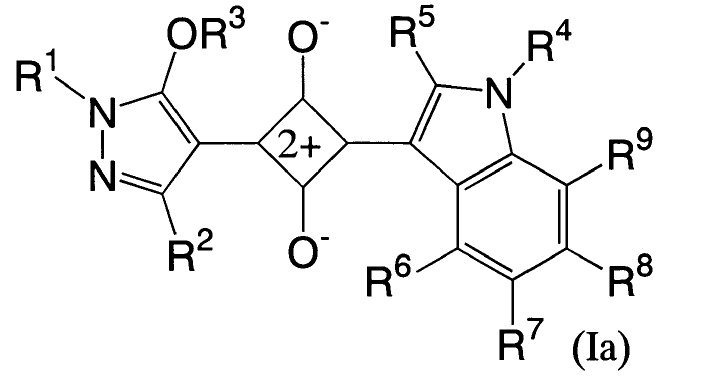 (wherein R¹, R², R³, R⁴, R⁵, R⁶, R⁷, R⁸, and R⁹ have the same definitions as described above, respectively).

3. An optical filter comprising a squarylium compound represented by General Formula (Ib): 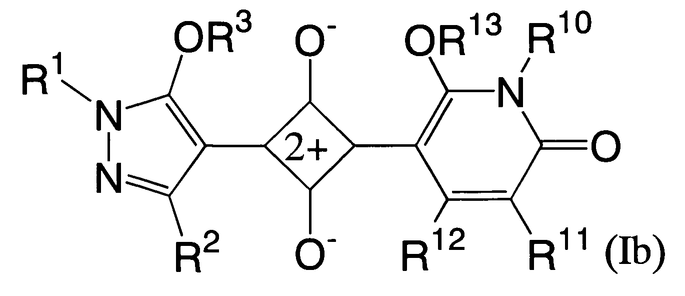 (wherein R¹, R², R³, R¹⁰, R¹¹, R¹² and R¹³ have the same definitions as described above, respectively).

4. An optical filter comprising a squarylium compound represented by General Formula (Ic): 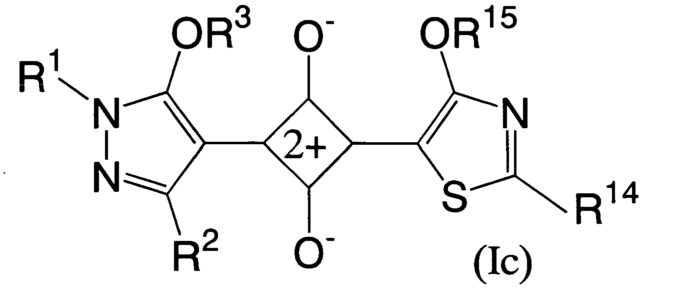 (wherein R¹, R², R³, R¹⁴ and R¹⁵ have the same definitions as described above, respectively).

5. The optical filter according to any one of Claims 1 to 4, which has an absorption maximum in a wavelength region of 530 nm to 580 nm.

6. The optical filter according to any one of Claims 1 to 5, wherein the optical filter is a filter for plasma display devices.

7. A squarylium compound represented by General Formula (I): 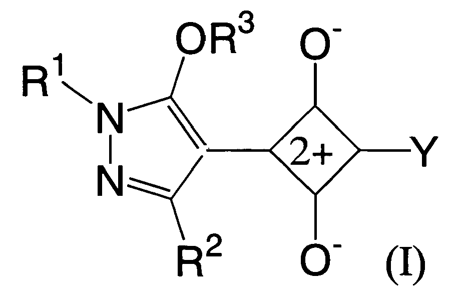 (wherein R¹, R², R³ and Y have the same definitions as described above, respectively).

8. A squarylium compound represented by General Formula (Ia): 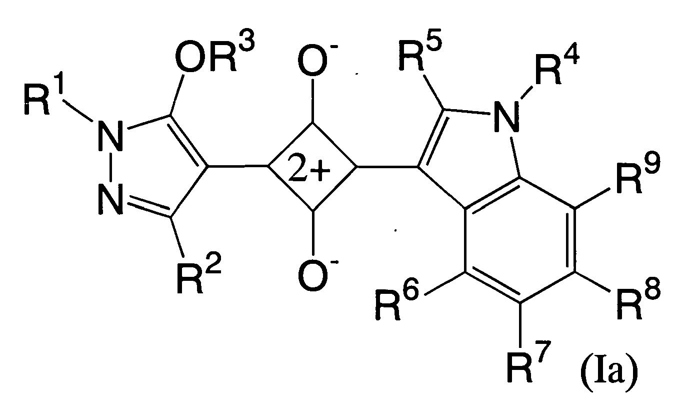 (wherein R¹, R², R³, R⁴, R⁵, R⁶, R⁷, R⁸ and R⁹ have the same definitions as described above, respectively).

9. A squarylium compound represented by General Formula (Ib): 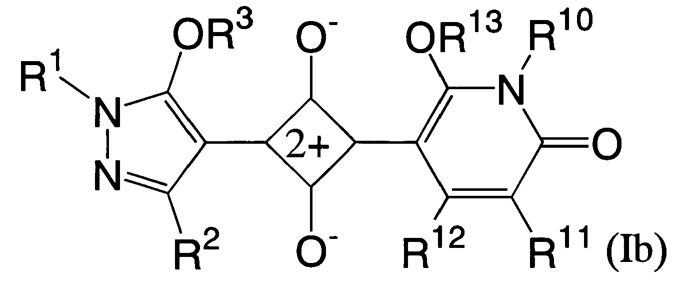 (wherein R¹, R², R³, R¹⁰, R¹¹, R¹², and R¹³ have the same definitions as described above, respectively).

10. A squarylium compound represented by General Formula (Ic): 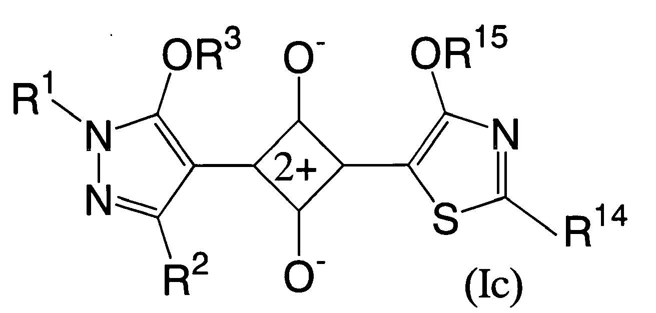 (wherein R¹, R², R³, R¹⁴ and R¹⁵ have the same definitions as described above, respectively).

11. The squarylium compound according to Claim 8, wherein R³ represents a hydrogen atom and R⁴ represents a hydrogen atom, an alkyl group or an alkoxyalkoxy-substituted alkyl group.

12. The squarylium compound according to Claim 9, wherein R³ and R¹³ each represents a hydrogen atom and R¹¹ represents a cyano group.

13. The squarylium compound according to Claim 10, wherein R³ represents a hydrogen atom, R¹⁴ represents an amino group optionally having substituent(s), or a heterocyclic group optionally having substituent(s), and R¹⁵ represents an alkyl group.

14. The squarylium compound according to any one of Claims 7 to 13, wherein R¹ represents a tert-butyl group or a phenyl group.
